# EUROPÄISCHE PATENTSCHRIFT

(11) **EP 0 844 896 B1**
(45) Veröffentlichungstag und Bekanntmachung des Hinweises auf die Patenterteilung: **22.10.2003**
(21) Anmeldenummer: 96931732.0
(22) Anmeldetag: 01.08.1996
(51) Int. Cl.: A61N 1/04

(54) **MIKROELEKTRODEN-ANORDNUNG**
MICRO-ELECTRODE ARRANGEMENT
SYSTEME DE MICRO-ELECTRODES

(30) Priorität: 10.08.1995 DE 19529371
(43) Veröffentlichungstag der Anmeldung: 03.06.1998
(60) Teilanmeldung: 03012355.8
(73) Patentinhaber: NMI Naturwissenschaftliches und Medizinisches Institut an der Universität Tübingen in Reutlingen, D-72762 Reutlingen (DE)
(72) Erfinder: NISCH, Wilfried, D-72072 Tübingen (DE)
(74) Vertreter: Witte, Alexander, Dr.-Ing.
(86) Internationale Anmeldenummer: DE9601428
(87) Internationale Veröffentlichungsnummer: WO97005922

(56) Entgegenhaltungen:
- DE-A- 3 813 838
- DE-A- 4 013 188
- US-A- 3 848 608
- US-A- 4 461 304
- US-A- 5 178 161

## Beschreibung

Die Erfindung betrifft die Verwendung einer Mikroelektroden-Anordnung zur ortsaufgelösten, insbesondere extrazellulären Ableitung elektrischer Zellpotentiale von Netzwerken biologischer Zellen.

Biologische Zellen oder Netzwerke aus biologischen Zellen wie z. B. Zellkulturen, Gewebeschnitte "in vitro" oder biologisches Gewebe "in vivo" werden in der Elektrophysiologie üblicherweise durch Glasmikroelektroden mit Elektrolytfüllung oder durch Metallmikroelektroden kontaktiert. Die Elektroden werden mittels eines sog. Mikromanipulators in eine Zelle eingestochen (intrazelluläres Verfahren), mit einer Zellmembran in dichten Kontakt gebracht (patch clamp - Verfahren) oder in die Nähe der Zellmembran gebracht (extrazelluläres Verfahren), so daß die Mikroelektroden elektrisch leitend über eine Elektrolytlösung mit den biologischen Zellen des Netzwerks verbunden ist. Der Nachteil dieser Kontaktier-Verfahren ist, daß nur eine oder mit großem Aufwand nur wenige Zellen gleichzeitig mit Mikroelektroden kontaktiert und infolgedessen keine Netzwerkeigenschaften untersucht werden können.

Aus diesem Grunde wurde in neuerer Zeit versucht, ein Netzwerk aus biologischen Zellen mittels Mikroelektroden, die auf ein Substrat (Träger) mit aus der Mikroelektronik bekannten Methoden aufgebracht und mikrostrukturiert sind, an vielen Stellen gleichzeitig zu kontaktieren, um elektrische Zellpotentiale extrazellulär ableiten oder die Zellen elektrisch stimulieren zu können. Dabei sollen die Mikroelektroden in möglichst hoher Dichte angeordnet sein, um eine hohe örtliche Auflösung zu erzielen. Desweiteren sollen die elektrischen Potentiale der Zellen möglichst gleichzeitig, also parallel, abgeleitet bzw. elektrische Potentiale zur Stimulation des Netzwerks gleichzeitig an dessen Zellen angelegt werden können, um eine hohe zeitliche Auflösung zu erreichen.

Dabei besteht allerdings das Problem, daß elektrische Leitungen von den einzelnen Mikroelektroden isoliert bis zu einer Meß- oder Stimulationselektronik oder dgl. geführt werden müssen. Die Vielzahl voneinander isolierter, paralleler Leitungen begrenzt die örtliche Auflösung der Mikroelektroden-Anordnung. Eine derartige Mikroelektroden-Anordnung ist bekannt aus der US-PS 4 461 304. Deren Substrat weist die Form einer Nadel auf, auf der Kontaktierelektroden an einer Nadelspitze beginnend mit Abstand voneinander in einer Linie aufgebracht sind. Die Kontaktierelektroden sind über Leiterbahnen elektrisch leitend mit Anschlußelektroden verbunden, die elektrisch leitend mit einem Meßgerät verbindbar sind. Die Leiterbahnen verlaufen parallel zueinander in Längsrichtung des nadelförmigen Substrats. Da die Leiterbahnen Abstand voneinander aufweisen müssen, um sie elektrisch voneinander zu isolieren, und durch die Anordnung der Kontaktierelektroden in einer Reihe, ist die Anzahl der Kontaktierelektroden auf wenige begrenzt.

Eine andere Möglichkeit ist, einen integrierten elektronischen Schalter für jede Mikroelektrode auf dem Substrat unterzubringen und die Mikroelektroden im Multiplexbetrieb einzeln oder in Gruppen zeitlich nacheinander mit der Meß- oder Stimulationselektronik zu verbinden (anzusteuern). Dies erfordert einen sehr hohen Aufwand an integrierter Schaltungstechnik (VLSI Technik) und verteuert dadurch die Mikroelektroden-Anordnung ganz erheblich. Des weiteren bleibt die örtliche Auflösung wegen der auf dem Substrat unterzubringenden elektronischen Schalter begrenzt. Darüber hinaus können die Mikroelektroden nicht mehr gleichzeitig, sondern nur einzeln oder in Gruppen nacheinander angesteuert werden, die Zeitauflösung der Ableitung oder Stimulation wird herabgesetzt. Weiterer Nachteil sind Störspannungen, die von den elektronischen Schaltern beim Schalten auf die Mikroelektroden und auf deren Anschlußleitungen übertragen werden können und das Meßsignal überlagern. Diese Störspannungen verschlechtern das Meßergebnis und das Signal/Rauschverhältnis. Die Störspannungen können das Meßsignal um ein Vielfaches übersteigen, weswegen ihr Abklingen nach dem Schalten abgewartet werden muß, bevor überhaupt gemessen oder stimuliert werden kann. Dadurch wird die Zeitauflösung der Mikroelektroden-Anordnung weiter herabgesetzt.

Die Anzahl der Mikroelektroden bekannter Mikroelektroden-Anordnungen ist infolgedessen begrenzt (weniger als 100 Mikroelektroden).

Der Erfindung liegt daher die Aufgabe zugrunde, eine Verwendung der eingangs genannten Art mit einer sehr großen Anzahl an Mikroelektroden zu schaffen, die durch kleine Abmessungen der Mikroelektroden und Abstände voneinander eine hohe Ortsauflösung und außerdem eine hohe zeitliche Auflösung ermöglicht.

Diese Aufgabe wird durch die Merkmale des Anspruchs 1 gelöst. Jede Mikroelektrode der erfindungsgemäß verwendeten Mikroelektroden-Anordnung weist eine Kontaktierelektrode, einen Anschluß für eine Meß- elektronik oder dgl., im folgenden als Anschlußelektrode bezeichnet, sowie ein lichtempfindliches Element auf.

Die Kontaktierelektrode ist über eine Elektrolytlösung in elektrisch leitenden Kontakt mit einer biologischen Zelle eines Netzwerks bringbar. Dies erfolgt vorzugsweise, indem die Mikroelektroden-Anordnung an ein Netzwerk biologischer Zellen heran und dadurch die Mikroelektroden in unmittelbare Nähe von Zellmembranen gebracht werden, also extrazellulär. Dabei besteht ein elektrischer Übergangswiderstand (impedanz) zwischen den Zellen und den Mikroelektroden.

Das lichtempfindliche Element, das bei Dunkelheit einen sehr hohen elektrischen Widerstand hat, der sich bei Auftreffen von Licht verringert (oder umgekehrt), ist zwischen den Kontaktierelektroden und den Anschlußelektroden angeordnet und dient als Schalter, der die Kontaktierelektrode von der Anschlußelektrode isoliert oder als ohmscher Widerstand mit der Anschlußelektrode verbindet. Betätigt wird dieser Schalter, indem Licht auf ihn, d. h. auf das lichtempfindliche Element, gerichtet wird. Somit ist jede Mikroelektrode für sich durch Licht ansteuerbar, die Mikroelektroden der erfindungsgemäß verwendeten Mikroelektroden-Anordnung sind lichtadressierbar.

Die Erfindung hat den Vorteil, daß die verwendeten Mikroelektroden sehr kleine Abmessungen aufweisen und sehr dicht beieinander anordenbar sind, so daß sich eine hohe örtliche Auflösung erzielen läßt. Weiterer Vorteil der Erfindung ist, daß die Mikroelektroden einzeln oder in Gruppen gleichzeitig, d. h. parallel ansteuerbar sind, was eine hohe zeitliche Auflösung ermöglicht. Weiterer Vorteil ist, daß durch die Ansteuerung mit Licht keine Störspannungen auftreten, die das Meßsignal überlagern und deren Abklingen vor einer Messung oder bis zu einer Stimulation abgewartet werden müßte.

Die Kontaktierelektroden, das lichtempfindliche Element und die Anschlußelektroden können in zwei oder drei Ebenen übereinander oder auch in eine Ebene nebeneinander auf einem Substrat angeordnet werden. Dabei ergibt die Anordnung in drei Ebenen übereinander die dichteste Anordnung der Mikroelektroden beieinander und damit die höchste örtliche Auflösung.

Zur Isolation der Kontaktierelektroden und der Anschlußelektroden der verschienen Mikroelektroden voneinander kann das lichtempfindliche Element dienen, das vorzugsweise wenn es nicht mit Licht beaufschlagt wird, also dunkel ist, elektrisch isoliert. Das lichtempfindliche Element ist in diesem Fall als für alle oder für Gruppen von Mikroelektroden gemeinsame, durchgehende Schicht ausgebildet, auf die örtlich auf die anzusteuernden Mikroelektroden begrenzt Licht gerichtet wird. In diesem Fall muß zur Ansteuerung mit Licht entweder die Kontaktierelektrode oder die Anschlußelektrode und das Substrat, auf das die Mikroelektroden aufgebracht sind, lichtdurchlässig sein.

Werden die lichtempfindlichen Elemente neben den Kontaktierelektroden oder neben den Anschlußelektroden angeordnet, so können die Kontaktierelektroden und die Anschlußelektroden lichtundurchlässig, aus demselben Material hergestellt und in einem Arbeitsgang auf das Substrat aufgebracht werden.

Die Anschlußelektroden aller oder von Gruppen der Mikroelektroden können zu einer gemeinsamen Anschlußelektrode vereinigt sein. Dadurch verringert sich die erforderliche Anzahl an Anschlußleitungen, jedoch können die Mikroelektroden nicht mehr parallel sondern nur seriell bzw. in Gruppen parallel angesteuert werden.

Zur Ansteuerung der Mikroelektroden ist bei einer Ausgestaltung der Erfindung eine Lichtfaseroptik vorgesehen, die vorzugsweise so viele Lichtfasern aufweist, wie die Anordnung Mikroelektroden umfaßt, so daß zu jeder Mikroelektrode eine Lichtfaser führt. Dabei können die Stirnenden der Lichtfasern, aus denen das Licht austritt, als Substrat für die Mikroelektroden dienen.

Bei einer Weiterbildung der Erfindung weist die Lichtfaseroptik eine Lichtquelle für jede Lichtfaser auf. Vorzugsweise sind die Lichtquellen zu einer Matrix zusammengefaßte Leuchtdioden.

Die erfindungsgemäß verwendete Mikroelektroden-Anordnung läßt sich zur Ableitung von Impulsen von Nervenzellen in Pflanzen oder Lebewesen implantieren.

Zur Ansteuerung bestimmter Mikroelektroden der erfindungsgemäßen Anordnung findet fokusiertes Licht, beispielsweise ein Laserstrahl Verwendung. Es können Muster aus Lichtpunkten, Lichtbalken oder dergleichen auf die Anordnung projiziert werden, um bestimmte Mikroelektroden gleichzeitig anzusteuern.

Die Erfindung wird nachfolgend anhand eines in der Zeichnung dargestellten Ausführungsbeispiels näher erläutert. Es zeigen:
- Figur 1: einen Schnitt durch eine erfindungsgemäß verwendete Mikroelektroden-Anordnung mit seriell (Figur 1a) bzw. parallel (Figur 1b) anzusteuernden Mikroelektroden;
- Figur 2: eine schematische Darstellung einer erfindungsgemäß verwendeten Mikroelektroden-Anordnung (Figur 2a seriell, Figur 2b parallel);
- Figur 3: eine Draufsicht auf eine erfindungsgemäße Mikroelektroden-Anordnung mit spaltenparallel geschalteten und zeilenparallel anzusteuernden Mikroelektroden; und
- Figur 4: einen Schnitt entlang Linie IV-IV in Figur 3.

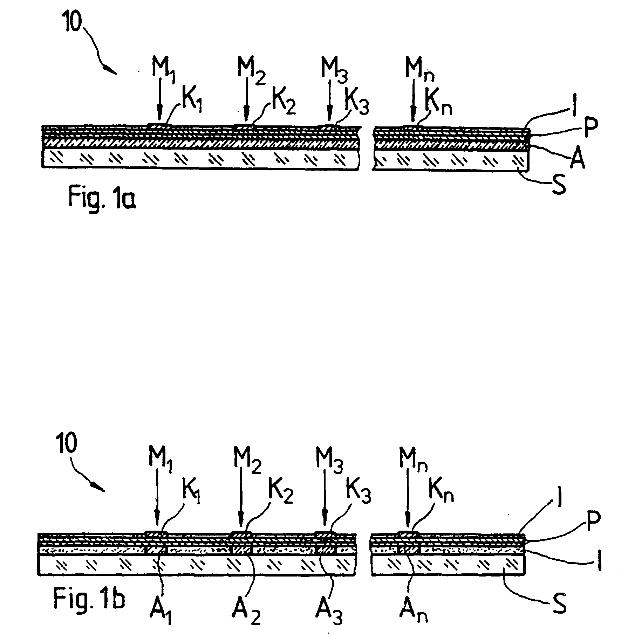

Die in Figuren 1a und b dargestellte, erfindungsgemäß verwendete Mikroelektroden-Anordnung 10 ist auf ein Substrat S aufgebracht. Das Substrat S besteht vorzugsweise aus einem lichtdurchlässigen Material, wie z. B. Glas oder Kunststoff. Es kann jedoch auch aus einem lichtundurchlässigen Material wie z. B. Keramik oder Silizium mit Oxidschichtisolator bestehen, die ansich aus der Mikroelektronik bekannt sind.

Die Mikroelektroden M₁ bis Mₙ umfassen Anschlußelektroden A, A₁ bis Aₙ, lichtempfindliche Elemente P und Kontaktierelektroden K₁-Kₙ, die in genannter Reihenfolgen in drei Ebenen übereinander als Dünnschichtelemente auf das Substrat S aufgebracht sind. Bei serieller Ansteuerung der Mikroelektroden M₁ bis Mₙ kann eine einzige, durchgehende Anschlußelektrode A für alle Mikroelektroden M₁ bis Mₙ gemeinsam auf das Substrat S aufgebracht sein (Figur 1a). Bei paralleler Ansteuerung weist jede Mikroelektrode M₁ bis Mₙ eine Anschlußelektrode A₁ bis Aₙ auf, die durch eine Isolatorschicht I voneinander getrennt sind. Die Isolatorschicht I ist in einer Ebene mit den Anschlußelektroden A₁ bis Aₙ auf das Substrat S aufgebracht.

Die lichtempfindlichen Elemente sind als durchgehende Schicht P für alle Mikroelektroden M₁ bis Mₙ gemeinsam auf die Anschlußelektroden A, A₁ bis Aₙ und ggf. die Isolierschicht I aufgebracht. Auf die die lichtempfindlichen Elemente bildende lichtempfindliche Schicht P sind die Kontaktierelektroden K₁ bis Kₙ aufgebracht, die sich bei paralleler Ansteuerung über den Anschlußelektroden A₁ bis Aₙ befinden. Die Kontaktierelektroden K₁ bis Kₙ sind ebenfalls mit einer Isolatorschicht I voneinander getrennt, die in einer Ebene mit den Kontaktiereleketroden K₁ bis Kₙ auf die lichtempfindliche Schicht P aufgebracht sind. Die Kontaktierelektroden K₁ bis Kₙ stehen geringfügig über ihre Isolatorschicht I vor.

Die als Dünnschichtelemente ausgebildeten Kontaktierelektroden K₁ bis Kₙ, lichtempflindlichen Elemente P und Anschlußelektroden A, A₁ bis Aₙ werden durch Aufdampfen, Sputtern oder PECVD (Plasma-Enhanced-Chemical-Vapor-Deposition) auf das Substrat S aufgebracht und mit photolhitografischen Methoden mikrostrukturiert.

Die Anschlußelektroden A, A₁ bis Aₙ bestehen aus einem elektrisch gut leitfähigen, vorzugsweise lichtdurchlässigen Material, wie z. B. Indiumzinnoxid (ITO) oder Zinkoxid (ZnO).

Die als durchgehende Schicht P ausgebildeten, lichtempfindlichen Elemente können als Dünnschicht-Fotowiderstände, Fotodioden mit PN- oder PIN-Übergang oder als Fototransistoren ausgeführt sein, die in Dünnschichttechnologie aus Materialien wie z. B. amorphem Silizium (Si), Cadmiumsulfid (CdS) oder Cadmiumselenid (CdSe) hergestellt sein können.

Die Kontaktierelektroden K₁ bis Kₙ bestehen vorzugsweise aus einem biokompatiblen, leitfähigen Material wie z. B. Gold (Au), Platin (Pt), Titan (Ti), Iridium (Ir) und sind durch die biokompatible Isolatorschicht I aus z. B. Siliziumoxid, Siliziumnitrid oder Polyimid voneinander isoliert. Die Kontaktierelektroden können auch aus lichtdurchlässigem Material, wie es für die Anschlußelektroden A, A₁ bis Aₙ Verwendung findet, hergestellt sein. Ebenso können die Anschlußelektroden A, A₁ bis Aₙ lichtundurchlässig aus demselben Material wie die Kontaktierelektroden K₁ bis Kₙ hergestellt sein.

Bei der in Figur 1a dargestellten Ausführungsform ist eine gemeinsame Leitung für alle Mikroelektroden M₁ bis Mₙ zum Anschluß an eine Meß- oder Stimulationselektronik oder dgl. an der gemeinsamen, durchgehend ausgebildeten Anschlußelektrode A, vorzugsweise in deren Randbereich, angebracht (nicht dargestellt). Bei der in Figur 1b dargestellten Ausführungsform der Erfindung mit voneinander isolierten Anschlußelektroden A₁ bis Aₙ weisen diese jeweils eigene Anschlußleitungen auf (nicht dargestellt).

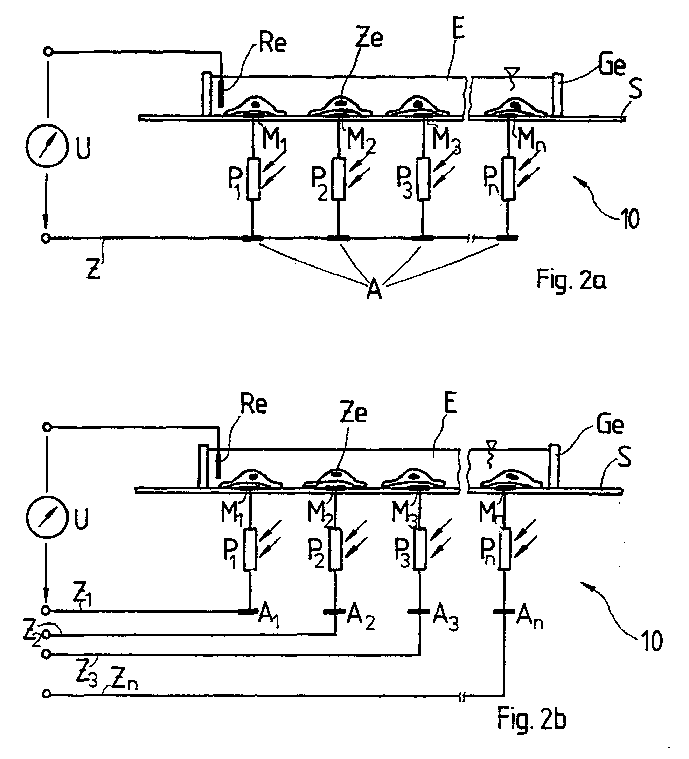

Die schematische Darstellung der Figuren 2a und b zeigt die Anwendung der Mikroelektroden-Anordnungen 10 aus Figuren 1a und b zur Ableitung elektrischer Zellpotentiale von Netzwerken biologischer Zellen Ze. Die biologischen Zellen Ze befinden sich in einem zylindrischen Kulturgefäß Ge in einem physiologischen Elektrolyten E. Den Boden des Kulturgefäßes Ge bildet das Substrat S mit der Mikroelektroden-Anordnung M₁ bis Mₙ aus Figuren 1a und b. Dabei befinden sich die in Figuren 2a und b nicht im einzelnen dargestellten Kontaktierelektroden K₁ bis Kₙ dicht an Zellmembranen der Zellen Ze und sind dadurch über den Elektrolyten elektrisch leitend mit jeweils einer Zelle Ze verbunden (extrazellulär), wobei ein elektrischer Widerstand (Impedanz) zwischen Zelle Ze und der Kontaktierelektrode K₁ bis Kₙ der jeweiligen Mikroelektrode M₁ bis Mₙ besteht.

In den physiologischen Elektrolyten E ist eine Referenzelektrode Re aus Metall getaucht, so daß ein elektrisches Potential an jeder gewünschten Stelle des Netzwerks biologischer Zellen Ze mit den Mikroelektroden M₁ bis Mₙ gemessen werden kann.

Die auf dem Substrat S aufgebrachten lichtempfindlichen Elemente P₁ bis Pₙ und Anschlußelektroden A, A₁ und Aₙ sind in Figuren 2a und b mit ihren Anschlußleitungen Z, Z₁ bis Zₙ in Form eines elektrischen Schaltbildes dargestellt.

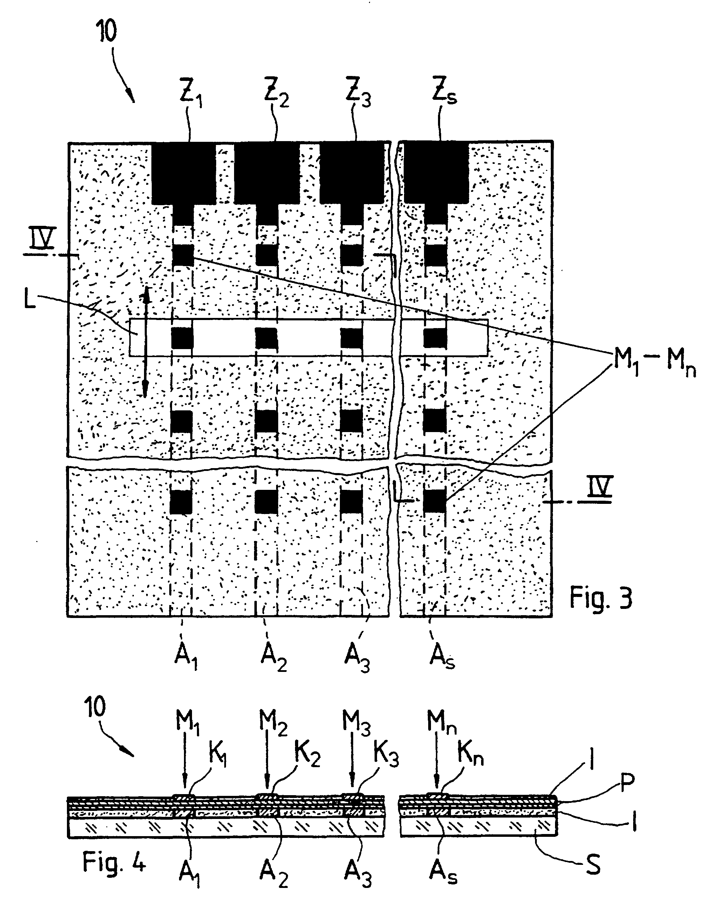

Die Figuren 3 und 4 zeigen eine erfindungsgemäße Mikroelektroden-Anordnung 10 mit spaltenparallel geschalteten Mikroelektroden M₁ bis Mₙ, wobei der Schnitt gemäß Figur 4 den Figuren 1a und b entspricht. Aufbau und Anordnung der Kontaktierelektroden K₁ bis Kₙ, die durch eine Isolatorschicht I voneinander isoliert sind, und die darunter liegende lichtempfindliche Dünnschicht P stimmt mit der oben beschriebenen, in Figuren 1a und b dargestellten Anordnung überein. In Figur 3 ist die matrixförmige Anordnung der Mikroelektroden M₁ bis Mₙ zu sehen. Anschlußelektroden A₁ bis Aₙ sind als parallele, in einer Spaltenrichtung durchgehende Leiterbahnen ausgebildet, die sich an einem Rand des Substrats S zu Kontaktierflächen Z₁ bis Zₛ vergrößern. An den Kontaktierflächen Z₁ bis Zₛ werden nicht dargestellte Anschlußkabel zum Anschluß der Mikroelektroden-Anordnung 10 an eine Meß- angelötet, angeschweißt oder auf sonstige, ansich bekannte Weise elektrisch leitend angebracht. Die Mikroelektroden M₁ bis Mₙ sind bei der Ausführungsform gemäß Figuren 3 und 4 zu je eine Spalte umfassenden Gruppen zusammengefaßt. Anstelle von Spalten können beispielsweise auch Kreise oder sonstige Gruppen von Mikroelektroden M₁ bis Mₙ zusammengefaßt werden.

Die Anschlußelektroden A₁ bis Aₛ sind durch eine Isolatorschicht I voneinander getrennt. Als Materialien für die Kontaktierelektroden K₁ bis Kₙ, die lichtempfindliche Schicht P, die Anschlußelektroden A₁ bis Aₙ, die Isolatorschichten I und das Substrat können die selben Materialien wie zu Figuren 1a und b aufgeführt Verwendung finden.

Bei der spaltenparallelen Schaltung der Mikroelektroden M₁ bis Mₙ kann jeweils nur eine Mikroelektrode M₁ bis Mₙ jeder Spalte angesteuert, d. h. mit ihr abgeleitet werden. Die Ansteuerung kann zeilenweise oder auch nach einem anderen Muster erfolgen.

Die Ansteuerung der Mikroelektroden-Anordnungen 10, die nachfolgend anhand Figur 3 erläutert wird, erfolgt mittels eines fokussierten oder geformten Lichtstrahls oder eines projizierten Lichtbildes, das beispielsweise unter Verwendung eines Lasers erzeugt oder mittels Glasfasern den Mikroelektroden M₁ bis Mₙ zugeführt wird. Zur Ansteuerung wird die lichtempfindliche Schicht P im Bereich einer oder mehrerer anzusteuernder Mikroelektroden M₁ bis Mₙ beleuchtet. Der beleuchtete Bereich bildet das lichtempfindliche Element der jeweiligen Mikroelektrode M₁ bis Mₙ. Der beleuchtete Bereich der lichtempfindlichen Schicht P wird elektrisch leitend, so daß die Kontaktierelektroden K₁ bis Kₙ der angesteuerten Mikroelektroden M₁ bis Mₙ elektrisch leitend mit der zugehörigen Anschlußelektrode A₁ bis Aₛ verbunden ist und das elektrische Potential einer in der Nähe der jeweiligen Mikroelektrode M₁ bis Mₙ befindlichen, biologischen Zelle (Figuren 2a und b) abgeleitet, d. h. gemessen werden kann.

Die Ansteuerung erfolgt entweder mittels Auflicht d. h. durch das Netzwerk biologischer Zellen hindurch von der Seite der Kontaktierelektroden K₁ bis Kₙ her. In diesem Fall müssen die Kontaktierelektroden K₁ bis Kₙ lichtdurchlässig oder seitlich neben den sie von ihrer Anschlußelektrode A₁ bis Aₛ trennenden, das lichtempfindliche Element bildenden lichtempfindlichen Schicht P angeordnet sein. Ebenso kann die Ansteuerung mit Durchlicht von der Seite des Substrats S her erfolgen. In diesem Fall muß das Substrat S und müssen die Anschlußelektroden A₁ bis Aₛ lichtdurchlässig oder neben der sie von den Kontaktierelektroden K₁ bis Kₙ trennenden, das lichtempfindliche Element bildenden lichtempfindlichen Schicht P angeordnet sein. Im unbeleuchteten Bereich isoliert die Dünnschicht P. Sie bildet also durch örtlich begrenzte Beleuchtung im Bereich einer Mikroelektrode M₁ bis Mₙ im beleuchteten Bereich das lichtempfindliche Element dieser Mikroelektrode M₁ bis Mₙ.

Bei Verwendung von amorphen Silizium werden bis zu fünf Zehnerpotenzen umfassende Widerstandsverhältnisse zwischen beleuchtet (hell) und unbeleuchtet (dunkel) erreicht. Bei einer Mikroelektrode M₁ bis Mₙ mit einer Fläche von 10 µm mal 10 µm und einer Dicke von 0,1 µm ergibt sich bei einer Dunkelleitfähigkeit von Sigma = 10⁻⁹ (Ohm x cm)⁻¹ ein Dunkelwiderstand von 10¹⁰ Ω und bei Lichtbestrahlung ein Hellwiderstand von 10⁵ Ω. Eine Kontaktierelektrode K₁ bis Kₙ hat bei der genannten Fläche von 10 µm x 10 µm durch das Elektrolyt E zur biologischen Zelle Ze einen Widerstand von etwa ebenfalls 10⁵ Ω, der durch die Helmholtz-Doppelschicht an der Grenzfläche Metall/Elektrolyt bestimmt wird. Es ergibt sich ein Gesamtübergangswiderstand von der biologischen Zelle Ze zur Anschlußelektrode A₁ bis Aₛ bei Lichtbestrahlung des lichtempfindlichen Elements P ein Widerstand von etwa 2 x 10⁵ Ω. Ihm gegenüber beträgt der Gesamtübergangswiderstand bei dunklem lichtempfindlichem Element P etwa 10¹⁰ Ω. Es ergibt sich ein gutes Kontakt/Trenn-Verhältnis durch die hell/dunkel-Tastung der Mikroelektroden M₁ bis Mₙ zu ihrer Ansteuerung.

Da der Abstand zwischen den Mikroelektroden M₁ bis Mₙ groß gegenüber der Schichtdicke der lichtempfindlichen Schicht P ist, kann auf eine Isolierung der von ihr gebildeten lichtempfindlichen Elemente voneinander verzichtet werden und diese als durchgehende Schicht P ausgeführt sein, wie es beschrieben und dargestellt ist. Die Ansteuerung der Mikroelektroden M₁ bis Mₙ erfolgt bei dem in Figur 3 dargestellten Ausführungsbeispiel der Erfindung mittels eines in Zeilenrichtung, also quer zu den Anschlußelektroden A₁ bis Aₛ verlaufenden Lichtbalkens L, der die lichtempfindlichen Elemente in. einer Zeile angeordneter Mikroelektroden M₁ bis Mₙ beleuchtet. Es werden also die Mikroelektroden M₁ bis Mₙ einer Zeile gleichzeitig angesteuert und die elektrischen Zellpotentiale der von diesen kontaktierten biologischen Zellen Ze über die Anschlußelektroden A₁ bis Aₛ abgeleitet. Der Lichtbalken L ist in Spaltenrichtung beweglich (Doppelpfeil in Figur 3). Die Ansteuerung kann selbstverständlich auch in verschiedenen Zeilen erfolgen, also nicht mittels eines Lichtbalkens, sondern mittels auf einzelne Mikroelektroden M₁ bis Mₙ gerichteter Lichtpunkte, wobei aus jeder Spalte nur eine Mikroelektrode M₁ bis Mₙ zu einem Zeitpunkt angesteuert werden kann. Ist der Abstand der Mikroelektroden M₁ bis Mₙ nicht ausreichend groß, so daß sich die Signale nebeneinander liegender Mikroelektroden M₁ bis Mₙ im vom Lichtbalken L beleuchteten und damit leitfähigen Bereich der lichtempfindlichen Schicht P gegenseitig beinflussen, so kann kein durchgehender Lichtbalken L zur Ansteuerung der Mikroelektroden M₁ bis Mₙ Verwendung finden, es muß vielmehr zwischen den Mikroelektroden M₁ bis Mₙ stets ein dunkler Bereich verbleiben oder aber eine zusätzliche Isolatorschicht zwischen den Anschlußelektroden A₁ bis Aₛ in der lichtempfindlichen Schicht P angebracht sein (nicht dargestellt).

Bei einer Mikroelektrodenfläche von 10 µm x 10 µm und bei 20 µm Elektrodenabstand ergeben sich bei beispielsweise 60 Spalten mit jeweils 60 Mikroelektroden insgesamt 3600 Mikroelektroden M₁ bis Mₙ auf einem Substratfeld mit einer Fläche von 1,8 mm x 1,8 mm.

Bei der Mikroelektroden-Anordnung kann die Ansteuerung der lichtempfindlichen Elemente gegebenenfalls auch mit einer Leuchtdiodenmatrix als Substrat oder durch ein projiziertes Lichtbild erfolgen.

## Patentansprüche

1. Verwendung einer Mikroelektroden-Anordnung zum ortsaufgelösten Ableiten elektrischer Zellpotentiale von Netzwerken biologischer Zellen, wobei die Mikroelektroden-Anordnung eine Vielzahl von Mikroelektroden aufweist und wobei jede Mikroelektrode (M₁ bis Mₙ) eine Kontaktierelektrode (K₁ bis Kₙ), die mit dem Netzwerk biologischer Zellen (Ze) in elektrischen Kontakt bringbar ist, eine Anschlußelektrode (A; A₁ bis Aₛ), die elektrisch leitend mit einem Meßgerät oder einer Meßelektronik verbindbar ist, und ein lichtempfindliches Element (P) aufweist, das zwischen der Kontaktierelektrode (K₁ bis Kₙ) und der Anschlußelektrode (A; A₁ bis Aₛ) angeordnet ist, wobei die Verwendung den Schritt umfaßt, daß die Mikroelektroden-Anordnung mit biologischen Zellen in vitro in Kontakt gebracht wird.

2. Verwendung nach Anspruch 1, **dadurch gekennzeichnet, daß** die Kontaktierelektrode (K₁ bis Kₙ) und/oder das lichtempfindliche Element (P) und/oder die Anschlußelektrode (A; A₁ bis Aₙ, A₁ bis Aₛ) Dünnschichtelemente sind.

3. Verwendung nach Anspruch 1 oder 2, **dadurch gekennzeichnet, daß** eine gemeinsame Anschlußelektrode (A; A₁ bis Aₛ) für alle Mikroelektroden (M₁ bis Mₙ) oder für eine Gruppe von Mikroelektroden (M₁ bis Mₙ) vorgesehen ist.

4. Verwendung nach einem der Ansprüche 1 bis 3, **dadurch gekennzeichnet, daß** das lichtempfindliche Element (P) durchgehend über den Bereich aller oder mehrerer Mikroelektroden (M₁ bis Mₙ) ausgebildet ist.

5. Verwendung nach einem der Ansprüche 1 bis 4, **dadurch gekennzeichnet, daß** eine Lichtfaseroptik zur Ansteuerung der Mikroelektroden (M₁ bis Mₙ) vorgesehen ist.

6. Verwendung nach Anspruch 5, **dadurch gekennzeichnet, daß** die Lichtfaseroptik eine Lichtfaser für jede Mikroelektrode (M₁ bis Mₙ) aufweist.

7. Verwendung nach Anspruch 6, **dadurch gekennzeichnet, daß** die Lichtfasern ein Substrat für die Mikroelektroden (M₁ bis Mₙ) bilden.

8. Verwendung nach Anspruch 6 oder 7, **dadurch gekennzeichnet, daß** die Lichtfaseroptik eine Lichtquelle für jede Lichtfaser aufweist.

9. Verwendung nach einem der Ansprüche 1 bis 8, **dadurch gekennzeichnet, daß** ein fokussierter Lichtstrahl örtlich begrenzt auf ein lichtempfindliches Element (P) einer oder mehrerer Mikroelektroden (M₁ bis Mₙ) gerichtet ist.

10. Verwendung nach Anspruch 1, **dadurch gekennzeichnet, daß** die Ansteuerung mit einer Leuchtdiodenmatrix als Substrat oder durch ein projiziertes Lichtbild erfolgt.

11. Verwendung nach einem der Ansprüche 1 bis 10, **dadurch gekennzeichnet, daß** das lichtempfindliche Element (P) als Dünnschicht-Photowiderstand ausgebildet ist.

12. Verwendung nach Anspruch 11, **dadurch gekennzeichnet, daß** das lichtempfindliche Element einen Dunkelwiderstand von näherungsweise 10¹⁰ Ω und einen Hellwiderstand von näherungsweise 10⁵ Ω aufweist.

13. Verwendung nach einem der Ansprüche 1 bis 12, **dadurch gekennzeichnet, daß** das lichtempfindliche Element (P) eine Dicke von 0,1 µm aufweist.

14. Verwendung nach einem der Ansprüche 1 bis 13, **dadurch gekennzeichnet, daß** das lichtempfindliche Element (P) amorphes Silizium aufweist.

## Claims

1. Use of a microelectrode arrangement for leaking, with local resolution, electrical cell potentials from networks of biological cells, said microelectrode arrangement comprising a multitude of microelectrodes, each microelectrode (M₁ to Mₙ) comprising a contacting electrode (K₁ to Kₙ) capable of being brought into electrical contact with the network of biological cells (Ze), a pad electrode (A; A₁ to Aₛ) which is connectable in an electrically conductive way to a measuring device or a measuring circuit, and a light-sensitive element (P), said use comprising the step that the microelectrode arrangement is being brought in vitro into contact with biological cells.

2. Use according to claim 1, **characterized in that** the contacting electrode (K₁ to Kₙ) and/or the light-sensitive element (P) and/or the pad electrode (A; A₁ to Aₙ, A₁ to Aₛ) are thin-film elements.

3. Use according to claim 1 or claim 2, **characterized in that** a common pad electrode (A; A₁ to Aₛ) for all microelectrodes (M₁ to Mₙ) or for a group of microelectrodes (M₁ to Mₙ) is provided.

4. Use according to anyone of claims 1 to 3, **characterized in that** the light-sensitive element (P) is constructed in a continuous way across the region of all or several microelectrodes (M₁ to Mₙ).

5. Use according to anyone of claims 1 to 4, **characterized in that** a fibreoptics for controlling the microelectrodes (M₁ to Mₙ) is provided.

6. Use according to claim 5, **characterized in that** the fibreoptics comprise an optic fibre for each microelectrode (M₁ to Mₙ).

7. Use according to claim 6, **characterized in that** the fibreoptics form a substrate for the microelectrodes (M₁ to Mₙ).

8. Use according to claim 6 or claim 7, **characterized in that** the fibreoptics comprise a light source for each optic fibre.

9. Use of anyone of claims 1 to 8, **characterized in that** a focused light beam is directed in a locally limited way onto a light-sensitive element (P) of one or several microelectrodes (M₁ to Mₙ).

10. Use according to claim 1, **characterized in that** controlling takes place with a light-emitting diode matrix as a substrate or by a projected light image.

11. Use according to anyone of claims 1 to 12, **characterized in that** the light-sensitive element (P) is designed as thin-film photo resistor.

12. Use according to claim 11, **characterized in that** the light-sensitive element comprises a dark resistance of about 10¹⁰ Ω and an illuminated resistance of about 10⁵ Ω.

13. Use according to anyone of claims 1 to 12, **characterized in that** the light-sensitive element has a thickness of 0,1 µm.

14. Use of anyone of claims 1 to 13, **characterized in that** the light-sensitive element (P) comprises amorphous silicon.

## Revendications

1. Utilisation d'un agencement de microélectrodes pour la dérivation par résolution locale de potentiels cellulaires électriques ou pour la stimulation électrique de réseaux de cellules biologiques, l'agencement de microélectrodes comportant une pluralité de microélectrodes, chaque microélectrode (M, à Mₙ) présentant une électrode de contact (K₁ à Kₙ), qui peut être amenée en contact électrique avec le réseau de cellules biologiques (Ze), une électrode de connexion (A ; A₁ à Aₛ), qui peut être connectée de manière électroconductrice à un appareil de mesure ou à une électronique de mesure, et un élément photosensible (P), qui est disposé entre l'électrode de contact (K₁ à Kₙ) et l'électrode de connexion (A ; A₁ à Aₛ), l'utilisation comprenant l'étape selon laquelle l'agencement de microélectrodes est amené en contact *in vitro* avec des cellules biologiques.

2. Utilisation selon la revendication 1, **caractérisée en ce que** l'électrode de contact (K₁ à Kₙ) et/ou l'élément photosensible (P) et/ou l'électrode de connexion (A ; A₁ à Aₙ, A₁ à Aₛ) sont des éléments à couche mince.

3. Utilisation selon la revendication 1 ou 2, **caractérisée en ce qu'**il est prévu une électrode de connexion (A ; A₁ à Aₛ) commune à toutes les microélectrodes (M₁ à Mₙ) ou à un groupe de microélectrodes (M₁ à Mₙ).

4. Utilisation selon l'une des revendications 1 à 3, **caractérisée en ce que** l'élément photosensible (P) est formé en continu sur la zone de toutes les microélectrodes (M₁ à Mₙ) ou de plusieurs d'entre elles.

5. Utilisation selon l'une des revendications 1 à 4, **caractérisée en ce qu'**il est prévu un système optique à fibres optiques pour l'excitation des microélectrodes (M₁ à Mₙ).

6. Utilisation selon la revendication 5, **caractérisée en ce que** le système optique à fibres optiques comporte une fibre photoconductrice pour chaque microélectrode (M₁ à Mₙ).

7. Utilisation selon la revendication 6, **caractérisée en ce que** les fibres optiques forment un substrat pour les microélectrodes (M₁ à Mₙ).

8. Utilisation selon la revendication 6 ou 7, **caractérisée en ce que** le système optique à fibres optiques comporte une source lumineuse pour chaque fibre optique.

9. Utilisation selon l'une des revendications 1 à 8, **caractérisée en ce qu'**un faisceau lumineux focalisé localement limité est dirigé sur un élément photosensible (P) d'une ou de plusieurs microélectrode(s) (M₁ à Mₙ).

10. Utilisation selon la revendication 1, **caractérisée en ce que** l'excitation se produit avec une matrice de diodes électroluminescentes en tant que substrat ou par une photographie projetée.

11. Utilisation selon l'une des revendications 1 à 10, **caractérisée en ce que** l'élément photo-sensible (P) est conformé en photorésistance à couche mince.

12. Utilisation selon la revendication 11, **caractérisée en ce que** l'élément photosensible possède une résistance à l'état sombre d'environ 10¹⁰ Ω et une résistance à l'état clair d'environ 10⁵ Ω.

13. Utilisation selon l'une des revendications 1 à 12, **caractérisée en ce que** l'élément photosensible (P) possède une épaisseur de 0,1 µm.

14. Utilisation selon l'une des revendications 1 à 13, **caractérisée en ce que** l'élément photosensible (P) comporte du silicium amorphe.
